# EUROPEAN PATENT SPECIFICATION

(11) **EP 3 463 054 B1**
(45) Date of publication and mention of the grant of the patent: **08.04.2020**
(21) Application number: 17742538.6
(22) Date of filing: 01.06.2017
(51) Int. Cl.: A61B 5/00, A61B 5/04, A61B 5/0488, A61B 5/0484

(54) **DEVICE, SYSTEM AND RELATING METHOD FOR THE QUANTITATIVE ASSESSMENT OF TASTE SENSITIVITY**
VORRICHTUNG, SYSTEM UND ZUGEHÖRIGES VERFAHREN ZUR QUANTITATIVEN BEURTEILUNG VON GESCHMACKSEMPFINDLICHKEIT
DISPOSITIF, SYSTÈME ET PROCÉDÉ ASSOCIÉ D'ÉVALUATION QUANTITATIVE DE LA SENSIBILITÉ GUSTATIVE

(30) Priority: 06.06.2016 IT UA20164123
(43) Date of publication of application: 10.04.2019
(73) Proprietor: Università Degli Studi di Cagliari, 09124 Cagliari (IT)
(72) Inventor: TOMASSINI BARBAROSSA, Iole, 09127 Cagliari (IT); CRNJAR, Roberto Massimo, 09045 Quartu S. Elena (CA) (IT); SOLLAI, Giorgia, 09121 Cagliari (IT); MELIS, Melania, 09040 Barrali (CA) (IT); PANI, Danilo, 09128 Cagliari (IT); COSSEDDU, Piero, 09125 Cagliari (IT); BONFIGLIO, Annalisa, 16040 Leivi (GE) (IT)
(74) Representative: Primiceri, Maria Vittoria
(86) International application number: PCT/IB2017/053254
(87) International publication number: WO 2017/212377

(56) References cited:
- JP-A- 2003 339 711
- JP-A- 2013 171 032
- US-A- 4 940 056

## Description

### Scope of the invention

The present invention relates to a device, system and relating method for the quantitative evaluation of the gustatory sensitivity.

### Background art

Human nutrition studies have been carried out on the involvement of the gustatory sensitivity in food preferences and in the nutritional and health status of individuals, reaching controversial conclusions.

This may be partly due to the fact that sensory analysis in humans is usually done through a multitude of psycho-physical methods that, although simple to apply, involve highly subjective assessments, and may also generate measurement errors that have been estimated to justify even 20% of the phenotypic variance.

Despite the above difficulties, psychometric tests are commonly used because they are the only methods currently in use that can be applied on populations of individuals at low cost and without causing discomfort or pain to the subject being examined.

Types of measurements of the degree of activation of the human gustatory system both at the central and peripheral level are known.

Known activation measures at the central level are performed using the Functional Magnetic Resonance Technique (RMF), which obviously cannot be applied to large numbers of subjects.

A measurement method at the peripheral level (Feldman et al. 2003; 2009) is known which uses a small vacuum-suction sensor applied to the tongue to detect the electrophysiological response to various gustatory stimuli of the human tongue. This method is also described in U.S. Pat. No. 4,940,056.

The use of this methodology is very limited. In fact, its implementation is considerably complicated, so it requires a lot of work and high costs, and its use is very invasive so as to prevent its application to large numbers of subjects.

The main drawbacks found in the existing solutions to detect the degree of activation of the gustatory system are the high costs and/or the difficulty of implementing the systems proposed in non-invasive disposable solutions that can be used simply on populations with large numbers of individuals.

There is therefore a need to find a non-invasive procedure that allows directly and objectively measuring the degree of activation of the gustatory system that is easy to use, reliable, applicable to large numbers of subjects and cost-effective.

### Summary of the invention

Therefore, the object of the present invention is a device, system and relating method for the quantitative evaluation of the gustatory sensitivity, aimed at overcoming the above drawbacks.

The object of the present invention is a system configured for quantitative evaluation of a person's gustatory sensitivity, characterized in that it comprises:
- a first electrode configured for direct application on the dorsal surface of the tip of the tongue, and comprising:
- a biocompatible metallic film area, having at least one central opening or microholes, said area being adapted to directly adhere to the dorsal surface of the tip of the tongue and being connected to an electric conductor, said at least one central opening or microholes being adapted to allow instillation of stimulating liquid on the tongue in order to trigger said quantitative evaluation of the gustatory sensitivity;
- a biocompatible and insulating plastic film, deposited around said silver film area, and wrapping a first part of said electric conductor, said insulating plastic film being adapted to adhere directly to the tongue;
- a second electrode of biocompatible metal configured for application in contact with the ventral surface of the person's tongue;
- a third electrode adapted to adhere to the person in an electrically neutral position;
- a biopotential measurement system, wherein said first electrode is connected to a positive terminal of the system, said second electrode is connected to a negative terminal of the system, said third electrode is connected to a reference terminal of the system, said measurement system providing a direct current coupling with said first and second electrodes;
- an electronic processing unit (5) adapted to receive the signals generated by said biopotential measurement system (4), and adapted to process said signals for analysing them and displaying their trend over time, for the purpose of obtaining the measurement of said quantitative evaluation of gustatory sensitivity.

It is also an object of the present invention an electrode configured to be applied directly to the dorsal surface of the person's tongue tip for the quantitative evaluation of the gustatory sensitivity.

A further object of the present invention is a process for making the electrode.

A further object of the present invention is a device configured for quantitative evaluation of a person's gustatory sensitivity.

A further object of the present invention is a process for the quantitative evaluation of a person's gustatory sensitivity using said system.

A particular object of the present invention is a device, system and method for the quantitative evaluation of the gustatory sensitivity, as better described in the claims which form an integral part of the present description.

### Brief description of the figures

Further objects and advantages of the present invention will become apparent from the following detailed description of an exemplary embodiment thereof (and of variants thereof) and with reference to the accompanying drawings, which are merely illustrative and non-limiting, in which:
figure 1 shows an embodiment example of the electrodes object of the present invention;
figure 2 shows an example of application of the electrodes to the person;
figure 3 shows a block diagram of the device;
figure 4 shows an example of the trend over time of the signal resulting from the use of the device;
figure 5 shows a flow chart of the method of use of the device;
figure 6 shows the mean values ± ES of a depolarization width (mV) (above) and the maximum value of the derivative (mV/s) (below), respectively, measured in super-taster, medium-taster, non-taster subjects and in homozygous subjects for the sensitive variant of the gustatory receptor PAV/PAV, heterozygote subjects PAV/AVI and homozygote subjects for the non-sensitive variant AVI/AVI;
figure 7 shows evaluation trends of human sensitivity of gustatory qualities referred to different substances.

The same reference numerals and letters in the figures identify the same elements or components.

### Detailed description

With reference to the figures, an object of the invention is a novel type of sensor (electrode) adapted to adhere to the dorsal surface of the tongue, exposing an area for depositing a sample of stimulating substance.

The sensing area of the sensor (electrode 2) is of biocompatible metal, preferably pure silver, evaporated on a thin film of a biocompatible plastic film such as polyimide, PET, Parylene, etc., of a thickness between 0.5 µm and 20 µm; each other part of the electrode is electrically insulated by passivation with a film (e.g. thickness of 2 µm) of biocompatible material, e.g. Parylene C. The thickness and type of materials ensure good adhesion of the electrode to the surface of the tongue without the use of adhesive substances, with good contact stability and good comfort for the subject.

As metal, gold, tungsten or others may for example be used for the electrode, said metal must form a non-polarizable interface with the liquid film covering the surface of the tongue.

The measurement procedure consists of a differential electrophysiological derivation, through the proposed electrode and a counter electrode (consisting, for example, of a silver ball positioned in contact with the ventral surface of the tongue), of the variations in the potential generated by the gustatory stimulation. The signal is acquired and transmitted to a PC for its real-time or quasi-real-time analysis.

The procedure allows the quantitative evaluation of the activation of the gustatory system through electrophysiological derivations of the bioelectric potentials generated by the gustatory stimulation. The potential detected on the tongue with this method is the result of the sum of the bioelectric responses of the stimulated gustatory cells.

An electrical signal is detected by differential measurement between the two passive electrodes mentioned above, positioned one (1) in contact with the ventral surface of the tongue and the other (2) on the dorsal surface of the tongue tip. A third electrode (3) adapted for the measurement of surface biopotentials (such as electrocardiographic, electromyographic, etc.), preferably disposable adhesive of solid gel Ag/AgCl, is used as a reference terminal *(signal ground)* for the measurement instrument, and is applied to the person in an electrically neutral position with respect to the phenomenon under observation (typically face or neck of the subject, not within the oral cavity).

As can be seen in the figures, the pair of electrodes for the differential electrophysiological derivation is heterogeneous in shape. The distal end of the electrode (1) consists of a biocompatible metal, such as deposited on Kapton or a silver filament (e.g.: Wire silver 0.50 mm 30 FT; WPISarasota, USA) (provided with an area with about 5 mm diameter), for example rolled up to form a small ball. The size is such to ensure a good surface for the electrical contact and makes the electrode (1) safe as regards possible injury or irritation of the sublingual mucous membrane. However, on the basis of these considerations, the size is not binding as is not the way in which this electrode is constructed. Other shapes may be used, provided that an equivalent result is obtained. Equivalently, an electrode of the same material as the electrode (2), of different shape, may be used, or even an electrode similar to that adopted on the dorsal surface of the tongue, with a different shape.

From the point of view of the measurement, it is preferable to use metals that form the biological element (especially with the liquid film covering it, like an electrolyte), a so-called "non-polarizable" contact. Also other metals or other pairs of materials of which one is metallic are fine, such as the silver/silver chloride pair. Other selection criteria are related to the biocompatibility and of course to the manufacturing simplicity and thus cost.

The electrode (2), which is the main component of the measurement system, is placed in contact with the dorsal surface of the tongue as described hereinafter. This electrode is made by depositing, for example through a high vacuum evaporation technique or sputtering, or printing techniques such as screen printing or inkjet, a biocompatible metal film, such as silver 50-100 nm thick, over a thin film of a biocompatible plastic film, such as polyimide, PET, Parylene etc., having a nominal thickness of between 0.5 µm and 20 µm. The extreme thinness of the film allows its perfect adhesion to the tongue when this is dried, using a piece of filter paper or other equivalent method, from the film of saliva normally present. An important aspect is that this adhesion is obtained directly to the tongue, without the use of adhesives or other substances. It should also be noted that polyimide is an inert material that is used in a variety of biomedical applications, also invasive (e.g.: Ordonez et al., Improved polyimide thin-film electrodes for neural implants, 2012) and that silver has long been used for manufacturing electrodes that come into contact with the mucosa (e.g.: Cobra® ET Tube Electrodes by Neurovision Medical Products™).

These aspects make the device particularly safe and at the same time simple, practical and low cost.

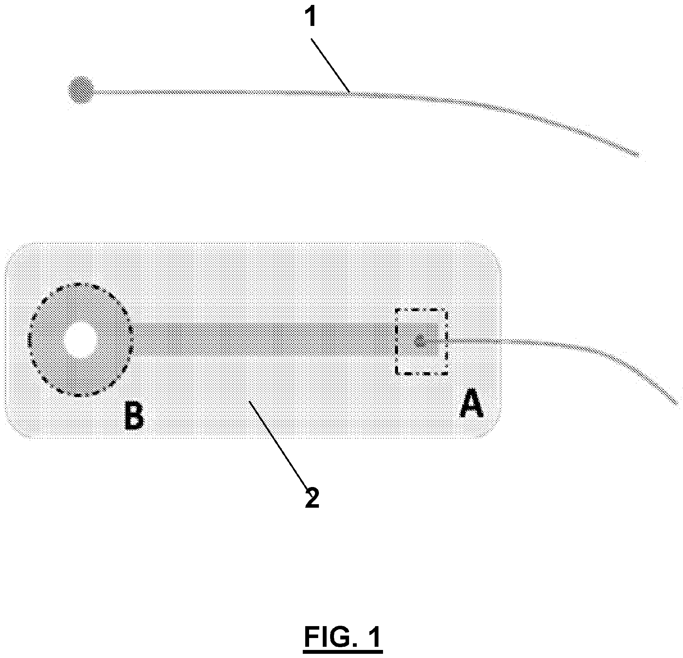

In the example described herein, the distal end of the electrode (2) has a sensing area, for example silver and ring-shaped. Typical sizes are 15 mm outer diameter (47 mm² area) and 6 mm inner diameter (19 mm² area). Figure 1 shows the overall area B. These dimensions represent a good compromise between the electrode selectivity (which derives from the limitation of the area affected by its application) and the generation of a signal (*interference pattern)* of regular shape. Another usable shape is microperforated area on which the stimulating fluid in drops is instilled, as described hereinafter.

The range of variation of these dimensional values is limited by anatomy (tongue dimensions) and by the sensing area of interest. When the electrode is positioned, the inner hole leaves a circular area of the front side of the dorsal surface of the tongue exposed, where the gustatory stimulation is applied, as described hereinafter, during the electrophysiological recordings. The selection of this position and this area is due to the fact that it provides reliable measurements of density of fungiform papillae that highly correlate with the total number of papillae on the tongue. The gustatory stimulations are made by instilling the stimulating fluid on the area. For example by placing a filter paper or equivalent absorbent material, comparable in size to the inner diameter of the circular crown of the electrode (2), impregnated with a gustatory system stimulating solution, in appropriate concentrations for the purpose and size of the electrode. For example as a stimulant, in the example described hereinafter, 30 µL of a concentration, for example 3.2 mM of 30 µL of 6-n-propylthiouracil (PROP) may be used.

Alternatively, solutions of sucrose, NaCl, citric acid, caffeine and umami may be used in concentrations sufficient to evoke the response of the gustatory cells (for the analysis of the 5 gustatory qualities). Oleic acid may also be used since now fat begins to be accepted as a sixth gustatory quality.

Alternatively, an electrode with identical features but different shape of the sensing surface may be used (while maintaining the area), i.e. with full circular sensing area with a diameter of between 6 mm and 15 mm, micro-perforated (with holes of diameter ranging between 0.8 mm and minimum of about 0.1 mm) so that the stimulating substance can be applied in drops on the micro-perforated part, or using a swab or equivalent method, penetrating through the holes and contacting the dorsal surface of the tongue, thus ensuring a direct contact with the papillae subjected to stimulation.

An isolated wire is welded (through the use of a conductive epoxy glue) to one end of the electrode (2) (area A in figure 1) and in proper use conditions, it remains outside the patient's mouth and not in contact with the tongue. This procedure allows a more reliable connection of the electrode to the measurement instrument.

A thin film (nominal thickness 2 µm) of insulating and biocompatible material, such as Parylene C, is further preferably deposited, as a protective layer, on the entire structure designed to cover both sides of the electrode with the exception of area B, which is the part of electrode that should be in electrical contact with the tongue.

The circular crown shape of the electrode is not binding, although considered preferred: other shapes may be used, such as regular polygons or ellipse, provided they have an equivalent surface, both as regards the overall area B, and for the empty interior.

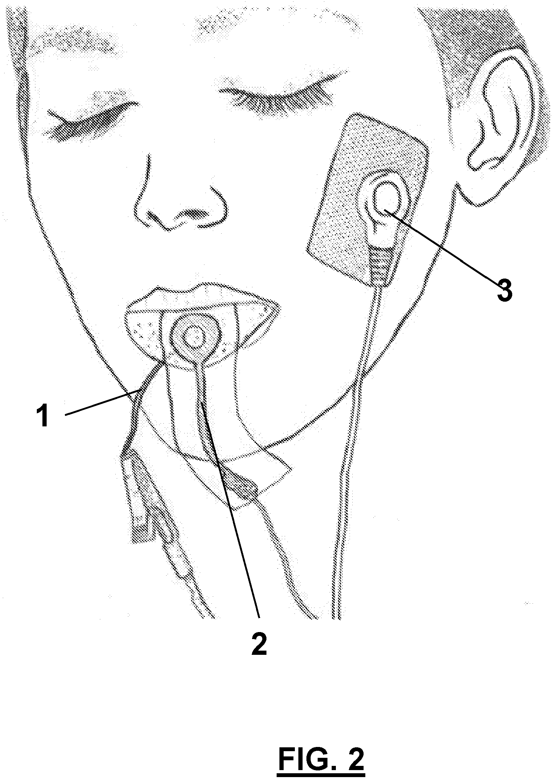

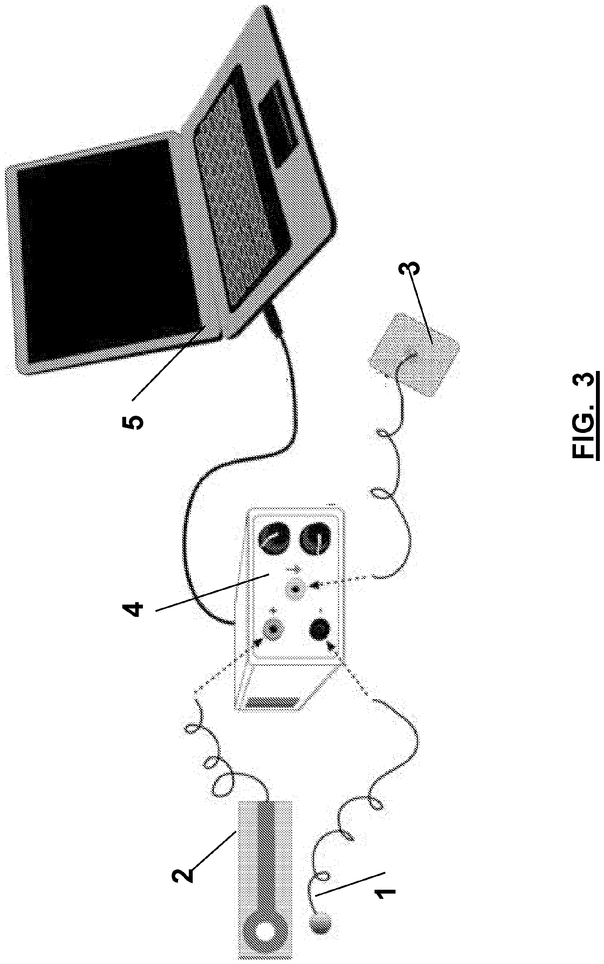

The electrodes, made as described and positioned as shown in figure 2, are connected to a biopotential measurement system (substantially known, custom or commercial, for example: Porti7 by TMSInternational) suitable for use on humans, for example as shown in figure 3. System (4) comprises a differential bioamplifier, known per se, and a digital data acquisition system known per se, allows the data acquisition, digitization and transmission, using an insulated wired connection (5), or via wireless connection, to an electronic processing unit, such as a PC (6), where the processing that is necessary for the real time or quasi-real-time display and analysis of the signal. The fundamental aspect is that the measurement device (4) ensures a direct coupling (DC), depending on the low frequency content of the signal detected by the electrodes. The measurement is differential, meaning that the electrodes (2) and (1) are connected to the positive and the negative terminal of a bioamplifier, respectively, while the electrode applied outside the mouth of the subject (3) is connected to the reference potential (*signal ground*) of the bioamplifier. No additional voltage component is applied to the electrodes by the measurement system, the only contribution of voltage is the one generated by the taste buds of the tongue, stimulated as described above.

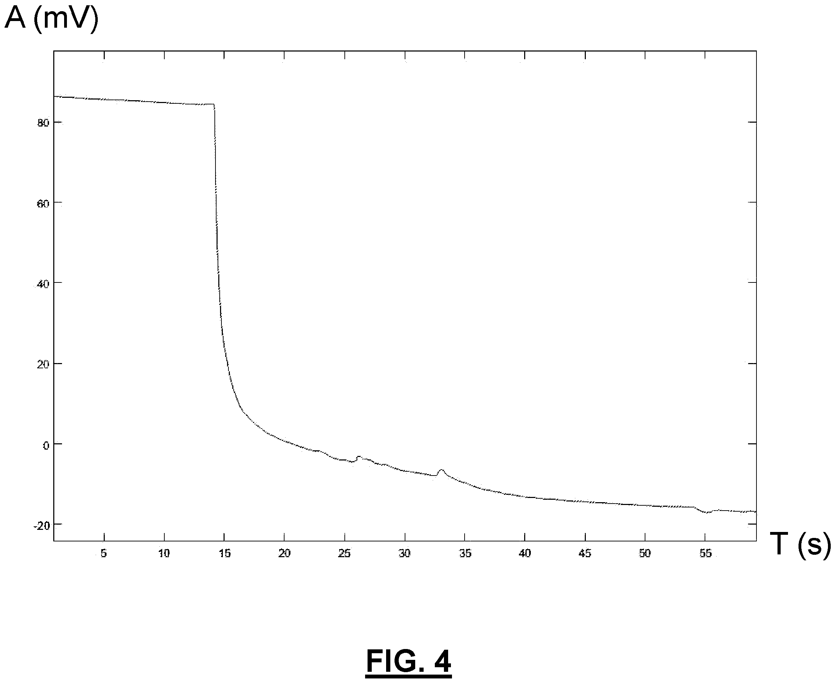

The signal analysis (figure 4) allows an objective quantification of the gustatory sensitivity to a given substance through the evaluation of the extent and dynamics of the biopotentials acquired. In the embodiment example described herein, the narrow-band signal, but that for reasons of time resolution should preferably be sampled at frequencies above 1000 Hz (alternatively, the signal may be acquired at a low sampling frequency - e.g.: 50-100 Hz - and then made a digital oversampling to improve the time resolution) is first pre-processed digitally in order to limit it in band below 10 Hz, then it is processed to extract features thereof useful for classification, i.e. the attribution of the subject on which the measurement is made to different classes depending on the sensitivity to the test substance. The features are of the morphological type, i.e. are related to the biopotential's shape and evolution dynamics. These include, but are not limited to, the amplitude of the depolarizing jump, the instantaneous and average speed of depolarization, the area under the depolarization curve, the product of this and its derivative (which is an example of "feature signal"), the depolarization end and half-depolarization times, the first and second derivative, etcetera, evaluated directly on the signal or a simplified version thereof stabilized after detrending, linear and non-linear filtering, fitting processes, etc. The extraction of features may require the delineation of the signal, i.e. the identification of fiducial points on it. Features may also be used such as the spectral content and time domain characteristics describable in the time-scale domain. The extracted features may be used to classify the samples using unsupervised (e.g. clustering) or supervised approaches (Support Vector machines (SVM), neural networks (MLP), etc.), where in the second case an appropriate process of labelling of samples and training has been made.

In an exemplary implementation, the signal is subjected to 10 Hz lowpass filtering, subjected to detrending (identifying the knee of the depolarization curve through joint analysis of the variability of the curve itself and its first derivative approximated by central difference at 5 points, and then estimating the straight line that best approximates to the least squares the stretch of 5 seconds before the knee itself, and subtracting it to all the signal), if the trend is reasonable, or there is no repolarization with stimulus still applied, and then segmented to detect the end of the depolarization. The resulting signal is simplified using a fitting with a sum of exponentials or a rational function (ratio of polynomials), and different features are extracted, including depolarization amplitude, the halving time of the area under the curve of the product between the signal and its first derivative (obtained analytically), the same area, the maximum of such a curve, the area under the depolarization curve, duration of depolarization, average depolarizing speed, amplitude of depolarization two seconds after its beginning, and the like.

Using 6-n-propylthiouracil (PROP) as the substance for gustatory stimulation, the results show that it is possible to automatically discriminate between subjects with high gustatory sensitivity (super-taster) and those with low or no gustatory sensitivity (non-taster) with respect to PROP with an accuracy greater than 95%, between non-tasters and tasters (super and medium, subjects with moderate gustatory sensitivity) with an accuracy greater than 80% and between super, non and medium, with an accuracy of about 65%, which is in line with the errors made by the current analytical methods, using an SVM or a KNN as a classifier.

Misclassifications are actually in line with experimental observations, in the sense that in the vast majority of cases it is not the classifier that goes wrong but the labeling of the data carried out on subjective perception scales by the subject under examination is inaccurate.

Once the algorithm has been defined, one can implement the functionalities described above even on a custom device which carries out the acquisition, digitization, processing and classification, without adding any PC to the measurement setup. Of course, in this case, the analysis algorithms should be implemented in the embedded system that will include not only the bioamplifier and the analog-to-digital converter, but also a microprocessor unit and outputs that allow viewing the signal and/or reporting the class of the subject under examination depending on the outcome of the classification made on the biopotential.

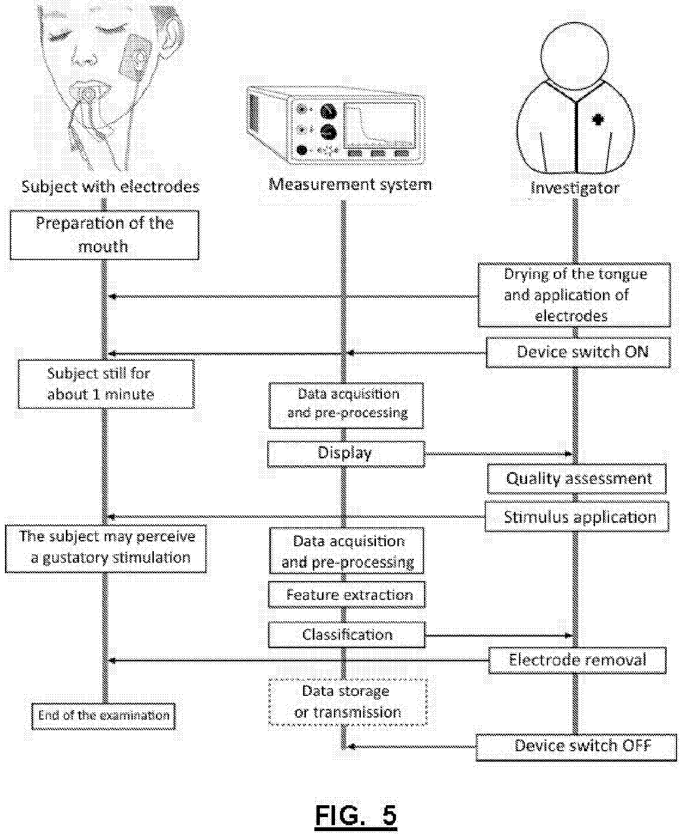

A flow chart related to the execution of a measurement is shown in figure 5. It is believed that the descriptions in the blocks of the figure are self-explanatory, and do not require further clarification.

The electrophysiological derivations obtained with this instrumental setup in response to gustatory stimulation with the stimulating substance used in the example described, namely 6-n-propylthiouracil (PROP), have been validated by comparing the amplitudes of signals with the intensity of the sensation evoked by the gustatory stimulation evaluated by psychophysical tests, with the phenotype and the genotype of the gustatory sensitivity to the PROP of known subjects. The PROP was selected for the validation of this instrumental setup as it was considered as a marker of the general gustatory sensitivity of a subject.

The stimulation with PROP evokes negative monophasic potentials, characterized by a quick initial depolarization followed by a slow decline. The amplitude of the signals correlates linearly with the density of fungiform papillae (r = 0504; P = 0.00057) and with the intensity of the perceived bitterness (r = 0741; P < 0.00001). The maximum value of the first derivative (used as an index of the depolarization speed) is also linearly correlated with the same parameters that define the phenotype PROP (with a papilla density of r = 0518; P = 0.00037, or perceived bitterness intensity r = 0511; P = 0.00053). The correlation coefficient r is a statistical index that indicates the linear correlation between two variables, and can take values of between -1 and 1. Positive values indicate the existence of a positive linear correlation; negative values indicate a negative correlation; the value 0 indicates no correlation. P indicates the significance level and in the case of linear correlation analysis between two variables, values of P < 0.05 indicate that the two variables are significantly related.

The amplitude of signals, as well as the maximum value of the first derivative, are associated with the status of the subjects for the sensitivity to PROP (One-way ANOVA: amplitude: F_{2.40} =12.836; P = 0.00005; maximum value of the first derivative: F_{2.40}= 7.6284; P = 0.00156), and with diplotypes *TAS2R38* (One-way ANOVA: amplitude: F_{2.40} =7.552; P = 0.00165; maximum value of the first derivative: F_{2.40} =3.699; P = 0.0333). High values of F and values of P < 0,05 indicate that the variables are significantly associated.

The Newman-Keuls post hoc test (per se known) shows that the signal amplitude values determined in non-tasters are smaller than those measured in medium-taster subjects (P = 0.0072), which in turn give lower values than super-tasters (P = 0.02776), and the values of the subjects with genotype AVI/AVI are smaller than those with genotypes PAV/PAV or AVI (PAV/P < 0.0128). Values of P<0.05 indicate significant variations.

In addition, the maximum first derivatives values in non-tasters or AVI/AVI are smaller respectively than those of super tasters (P = 0.00049; according to the Duncan test per se known), or those with at least one taster variant (PAV) in the TAS2R38 gene (P ≤ 0.0317; Newman-Keuls test).

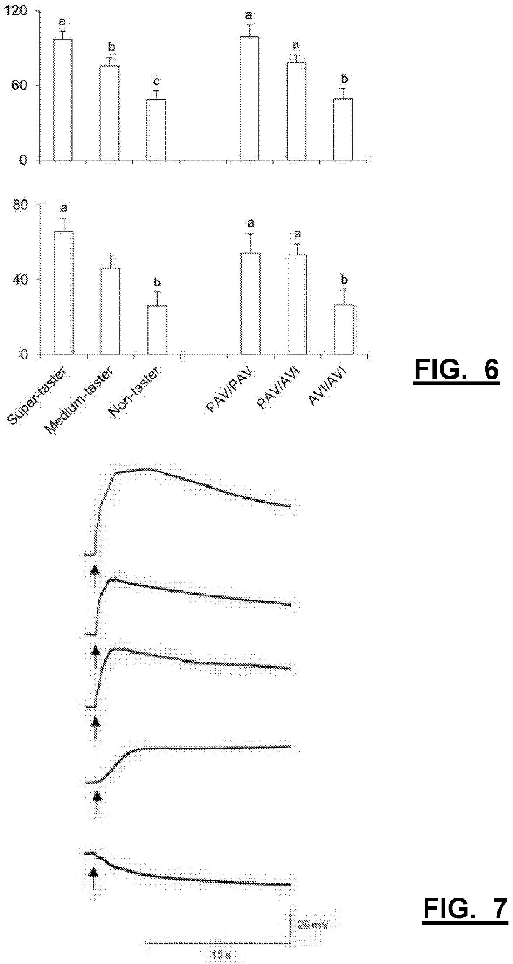

See figure 6, which shows the mean ± ES values of a depolarization width (mV) (above) and the maximum value of the derivative (mV/s) (below), respectively, measured in super-tasters (n=15), medium-tasters (n=15), non-tasters (n=13), and in subjects with genotype PAV/PAV (n=7), PAV/AVI (n=25), AVI/AVI (n=11). Bars with different letters are statistically different from each other by the Newman-Keuls test, subsequent to the one way ANOVA test (p ≤ 0.0317), and the value of p indicates the level of significance.

The bars with different letters are statistically different average values by the statistical test used.

ES is the standard error indicating the extent the values deviate from the mean.

These results suggest that the stimulation with the bitter taste of PROP evokes negative monophasic potentials, which represent the measurement of the sum of voltage variations that result from the response of taste cells stimulated, as shown by the direct and linear correlation found between the amplitude of the depolarization evoked by stimulation with PROP and the density of fungiform papillae measured in the same area of the tongue where the PROP stimulation was applied. On the other hand, the direct and linear correlation found between the amplitude and the depolarization speed, and the intensity of the perceived bitterness during stimulation with PROP, indicate that the bio-electrical measurements made are fully compatible with common current psyco-physical observations in the gustatory sensory evaluation on humans. Moreover, the direct relationship between the amplitudes of signals, as well as the depolarization speed, and the parameters that define the genotype and phenotype of sensitivity to PROP show that the method described herein is a simple, highly reliable, and mildly non-invasive technology for the quantitative measurement of gustatory function, which is not influenced by subjective factors that may lead to confusion.

The system may also be applied to the evaluation of human sensitivity to other gustatory qualities, as shown in figure 7, which shows the trends achieved, respectively (from top to bottom) with: 5.2 mM citric acid; 6.7 mM caffeine, 100 mM sucrose, 80 mM L-glutamate, 100 mM NaCl. The registration is carried out in a subject in response to 30 µL of solution of each stimulus for about 15 seconds.

Embodiment variants of the non-limiting example described are possible without departing from the scope of protection of the present invention, comprising all the equivalent embodiments for a man skilled in the art.

The elements and the features shown in the different preferred embodiments may be combined without departing from the scope of protection of the present invention.

The advantages resulting from the application of the present invention are clear.

The innovative aspect of the present invention is the fact of implementing a process that uses easy-to-use, low-cost disposable electrodes, whereby a passive, quantitative, objective, non-invasive and reliable measurement of the peripheral gustatory system activation degree of the subject is obtained.

From the above description, the man skilled in the art is able to implement the object of the invention without introducing any further construction details.

## Claims

1. Electrode (2) configured for direct application onto the dorsal surface of the tip of the tongue, and for allowing a quantitative evaluation of a person's gustatory sensitivity, **characterized in that** it comprises:
• a biocompatible metallic film area, having at least one central opening or microholes, said area being adapted to directly adhere to the dorsal surface of the tip of the tongue and being connected to an electric conductor, said at least one central opening or microholes being adapted to allow instillation of stimulating liquid on the tongue in order to trigger said quantitative evaluation of the gustatory sensitivity;
• a biocompatible and insulating plastic film, deposited around said biocompatible metallic film area, and wrapping a first part of said electric conductor, said insulating plastic film being adapted to adhere directly to the tongue.

2. Electrode (2) according to claim 1, wherein:
- said area made of biocompatible metallic film is shaped like a circular ring having an outside diameter of 15 mm and an inside diameter of 6 mm, with a thickness of 50-100 nm, or is shaped like an ellipse or a regular polygon, or is made of biocompatible metal comprising microholes, with an area equal to that of said circular shape and with the same thickness;
- said biocompatible insulating plastic film is Parylene C with a nominal thickness of 2 pm.

3. Electrode (2) according to claim 2, wherein said biocompatible metallic film is pure silver, or gold, or tungsten, or silver/silver chloride.

4. Process for manufacturing an electrode according to claim 1 or 2 or 3, **characterized in that** it comprises the steps of:
- depositing, by high-vacuum evaporation or sputtering or ink-jet printing or screen printing, said biocompatible metallic film area onto a biocompatible insulating plastic film, such as polyimide, PET, Parylene, having a thickness in the range of 0.5 pm to 20 pm, so at to wrap also said first part of electric conductor;
- applying, by passivation, said biocompatible plastic film around said biocompatible metallic film area and to a first part of said electric conductor.

5. Device configured for quantitative evaluation of a person's gustatory sensitivity, **characterized in that** it comprises:
- a first electrode (2) according to any one of claims 1-3;
- a second electrode (1) of biocompatible metal configured for application in contact with the ventral surface of the person's tongue;
- a third electrode (3) adapted to adhere to the person in an electrically neutral position.

6. Device according to claim 5, wherein said biocompatible metal in said second electrode (1) is a wound silver filament having a diameter of 5 mm.

7. System configured for quantitative evaluation of a person's gustatory sensitivity, **characterized in that** it comprises:
- the device according to any one of claims 5-6;
- a biopotential measurement system (4), wherein said first electrode is connected to a positive terminal of the system, said second electrode is connected to a negative terminal of the system, said third electrode is connected to a reference terminal of the system, said measurement system providing a direct current coupling with said first and second electrodes;
- an electronic processing unit (5) adapted to receive the signals generated by said biopotential measurement system (4), and adapted to process said signals for analysing them and displaying their trend over time, for the purpose of obtaining the measurement of said quantitative evaluation of gustatory sensitivity.

8. Process for quantitative evaluation of a person's gustatory sensitivity, using a system for quantitative evaluation of a person's gustatory sensitivity according to claim 7, **characterized in that** it comprises the steps of:
- sampling the signal generated by said biopotential measurement system (4) at frequencies higher than 1,000 Hz, or acquiring the signal at low-frequency sampling- e.g. 50-100 Hz - and then performing a digital oversampling in order to improve the time resolution,
- limiting the band of the signal generated by said biopotential measurement system (4) below 10 Hz,
- processing said band-limited signal in order to extract features useful for classification purposes, i.e. for assigning the person to different classes as a function of his/her sensitivity to said stimulating liquid, thereby obtaining said quantitative evaluation of the gustatory sensitivity;
- said features being of the morphological type and being related to the biopotential's shape and evolution dynamics.

## Patentansprüche

1. Elektrode (2), zum direkten Aufbringen auf die Rückenfläche der Zungenspitze und zur quantitativen Beurteilung der Geschmacksempfindlichkeit einer Person konfiguriert, **dadurch gekennzeichnet, dass** sie umfasst:
- einen Bereich aus biokompatiblem Metallfilm mit mindestens einer zentralen Öffnung oder Mikrolöchern, wobei der Bereich derart angepasst ist, dass er direkt an der Rückenfläche der Zungenspitze haften kann und mit einem elektrischen Leiter verbunden ist, wobei die mindestens eine mittlere Öffnung oder die Mikrolöcher derart geeignet sind, dass sie die Instillation einer anregenden Flüssigkeit auf der Zunge ermöglicht, um die genannte quantitative Bewertung der Geschmacksempfindlichkeit auszulösen;
- eine biokompatible und isolierende Kunststofffolie, die um den Bereich aus biokompatiblem Metallfilm herum aufgebracht ist und einen ersten Teil des elektrischen Leiters umhüllt, wobei die isolierende Kunststofffolie direkt an der Zunge haften kann.

2. Elektrode (2) nach Anspruch 1, wobei:
- der Bereich aus biokompatiblem Metallfilm entweder die Form eines Kreisrings mit einem Außendurchmesser von 15 mm und einem Innendurchmesser von 6 mm mit einer Dicke von 50-100 nm, oder die Form einer Ellipse oder eines regelmäßigen Polygons aufweist, oder aus einem biokompatiblem Metall hergestellt ist, das Mikrolöcher mit einer Fläche aufweist, die der der kreisförmigen Form entspricht und die gleiche Dicke aufweist;
- die biokompatible isolierende Kunststofffolie ist Parylene C mit einer Nenndicke von 2 pm.

3. Elektrode (2) nach Anspruch 2, wobei der genannte biokompatible Metallfilm ist reines Silber oder Gold oder Wolfram oder Silber/Silberchlorid.

4. Verfahren zur Herstellung einer Elektrode nach Anspruch 1 oder 2 oder 3, **dadurch gekennzeichnet, dass** es die folgenden Schritte umfasst:
- Hinterlegung, durch Verdampfung unter Abscheidung von Hochvakuum oder Kathodenzerstäubung oder Tintenstrahldruck oder Siebdruck, der biokompatiblen Metallfilmfläche aus einem biokompatiblen Plastikfilmisolator, wie beispielsweise aus Polyimid, PET, Parylen, mit einer Dicke zwischen 0,5 pm und 20 pm, damit auch der genannte erster Abschnitt des elektrischen Leiters eingewickelt wird;
- Aufbringung der biokompatiblen Kunststofffolie durch Passivierung um den genannten Bereich des biokompatiblen Metallfilms und auf einen ersten Teil des elektrischen Leiters.

5. Vorrichtung zur quantitativen Beurteilung der Geschmacksempfindlichkeit einer Person, **dadurch gekennzeichnet, dass** sie umfasst:
- eine erste Elektrode (2) nach einem der Ansprüche 1-3;
- eine zweite Elektrode (1) aus einem biokompatiblen Metall, konfiguriert für den Einsatz in Kontakt mit der ventralen Teil der Zunge der Person;
- eine dritte Elektrode (3), die angepasst ist, um an der Person in einer elektrisch neutralen Position zu haften.

6. Vorrichtung nach Anspruch 5, wobei das biokompatible Metall in der zweiten Elektrode (1) ein gewickeltes Silberfilament mit einem Durchmesser von 5 mm ist.

7. System zur quantitativen Bewertung der Geschmackssensitivität einer Person, **dadurch gekennzeichnet, dass** es umfasst:
- die Vorrichtung nach einem der Ansprüche 5-6;
- ein Biopotential-Messesystem (4), bei dem die erste Elektrode mit einem positiven Terminal des Systems verbunden ist, wobei die zweite Elektrode mit einem negativen Terminal des Systems verbunden ist, und die dritte Elektrode mit einem Referenzterminal des Systems verbunden ist, wobei das Messesystem eine Gleichstromkopplung mit der ersten und der zweiten Elektrode ermöglicht;
- eine elektronische Verarbeitungseinheit (5), die angepasst ist, um die von dem Biopotential-Messesystem (4) erzeugten Signale zu empfangen, und angepasst ist, um die Signale für ihre Analyse und für die Anzeige ihres zeitlichen Trends zu verarbeiten, um die Messung der genannten quantitativen Bewertung der Geschmacksempfindlichkeit zu erreichen.

8. Verfahren zur quantitativen Beurteilung der Geschmacksempfindlichkeit einer Person unter Verwendung eines Systems zur quantitativen Beurteilung der Geschmacksempfindlichkeit einer Person nach Anspruch 7, **dadurch gekennzeichnet, dass** es die folgenden Schritte umfasst:
- Abtastung des durch dem Biopotential-Messesystem (4) erzeugten Signals bei Frequenzen größer als 1000 Hz oder Erwerbung des Signals mit einer Probe bei einer niedrigen Frequenz, beispielsweise 50-100 Hz und dann Ausführen eines digitalen Überabtastung, zur Verbesserung der zeitlichen Auflösung,
- Begrenzung des Bandes des durch das Biopotential-Messesystem (4) erzeugten Signals unter 10 Hz,
- Verarbeitung des begrenzten Bandsignals, um nützliche Eigenschaften für Klassifizierungszwecke zu extrahieren, das heißt, um die Person gemäß ihrer Empfindlichkeit für die stimulierende Flüssigkeit zu verschiedenen Klassen zuzuordnen, wodurch die quantitative Bewertung des Geschmacksempfindlichkeit erhalten wird;
- die genannten Eigenschaften aus morphologischen Typ bestehen und sich in Bezug auf die Form und die dynamische Entwicklung des Biopotentials behalten.

## Revendications

1. Electrode (2) configurée pour une application directe sur la surface dorsale de la pointe de la langue, et pour permettre une évaluation quantitative de la sensibilité gustative d'une personne, **caractérisé en ce qu'**il comprend:
- une zone de film métallique biocompatible, ayant au moins une ouverture centrale ou des micro-trous, ladite zone étant adaptée pour adhérer directement à la surface dorsale de la pointe de la langue et étant connectée à un conducteur électrique, ladite au moins une ouverture centrale ou des micro-trous étant adaptée pour permettre l'instillation d'un liquide stimulant sur la langue afin de déclencher ladite évaluation quantitative de la sensibilité gustative;
- un film plastique biocompatible et isolant, déposé autour de ladite zone de film métallique biocompatible, et enveloppant une première partie dudit conducteur électrique, ledit film plastique isolant étant adapté pour adhérer directement à la langue.

2. Electrode (2) selon la revendication 1, dans laquelle:
- ladite zone en film métallique biocompatible est en forme d'anneau circulaire avec un diamètre extérieur de 15 mm et un diamètre intérieur de 6 mm, avec une épaisseur de 50-100 nm, ou en forme d'une ellipse ou d'un polygone régulier, ou en métal biocompatible comportant des micro-trous, de surface égale à celle de ladite forme circulaire et du même épaisseur;
- ledit film plastique isolant biocompatible est du Parylène C avec une épaisseur nominale de 2 pm.

3. Electrode (2) selon la revendication 2, dans lequel ledit film métallique biocompatible est de l'argent pur, ou de l'or, ou du tungstène, ou de l'argent/chlorure d'argent.

4. Procédé de production d'une électrode selon la revendication 1 ou 2 ou 3, **caractérisé en ce qu'**il comprend les étapes de:
- déposer, par évaporation sous haut vide ou pulvérisation cathodique ou impression par jet d'encre ou sérigraphie, ladite zone de film métallique biocompatible sur un film plastique isolant biocompatible, tel que de la polyimide, PET, Parylène, ayant une épaisseur comprise entre 0,5 pm et 20 pm, donc à envelopper également ladite première partie de conducteur électrique;
- appliquer, par passivation, ledit film plastique biocompatible autour de ladite zone de film métallique biocompatible et sur une première partie dudit conducteur électrique.

5. Dispositif configuré pour une évaluation quantitative de la sensibilité gustative d'une personne, **caractérisé en ce qu'**il comprend:
- une première électrode (2) selon l'une quelconque des revendications 1-3 ;
- une deuxième électrode (1) métallique biocompatible configurée pour une application en contact avec la surface ventrale de la langue de la personne;
- une troisième électrode (3) adaptée pour adhérer à la personne dans une position électriquement neutre.

6. Dispositif selon la revendication 5, dans lequel ledit métal biocompatible dans ledit deuxième électrode (1) est un filament d'argent enroulé avec un diamètre de 5 mm.

7. Système configuré pour évaluation quantitative de la sensibilité gustative d'une personne, **caractérisé en ce qu'**il comprend :
- le dispositif selon l'une quelconque des revendications 5-6 ;
- un système de mesure bio-potentiel (4) dans lequel ladite première électrode est connectée à un terminal positif du système, ladite deuxième électrode étant connectée à un terminal négatif du système, ladite troisième électrode étant connectée à un terminal de référence du système, ledit système de mesure fournissant un accouplement à courant directe avec lesdites première et deuxième électrodes;
- une unité (5) d'élaboration électronique adaptée pour recevoir les signaux générés par ledit système (4) de mesure bio-potentiel, et adapté pour élaborer lesdits signaux pour leur analyse et pour la visualisation de leur tendance dans le temps, afin d'obtenir la mesure de ladite évaluation quantitative de la sensibilité gustative.

8. Procédé pour une évaluation quantitative de la sensibilité gustative d'une personne, en utilisant un système pour l'évaluation quantitative de la sensibilité gustative d'une personne selon la revendication 7, **caractérisé en ce qu'**il comprend les étapes suivantes:
- échantillonner le signal généré par un système de mesure bio-potentiel (4) à des fréquences supérieures à 1000 Hz ou en acquérant le signal avec un échantillonnage à basse fréquence, par exemple de 50-100 Hz et puis en effectuant un sur-échantillonnage digital afin d'améliorer la résolution temporelle,
- limiter la bande du signal généré par ledit système de mesure bio-potentiel (4) en dessous de 10 Hz,
- élaborer ledit signal à bande limitée afin d'extraire des caractéristiques utiles à des fins de classification, c'est-à-dire pour affecter la personne à différentes classes en fonction de sa sensibilité audit liquide stimulant, obtenant ainsi ladite évaluation quantitative de la sensibilité gustative;
- lesdites caractéristiques étant de type morphologique et liées à la forme et à la dynamique d'évolution du bio-potentiel.
